# EUROPÄISCHE PATENTSCHRIFT

(11) **EP 0 700 679 B1**
(45) Veröffentlichungstag und Bekanntmachung des Hinweises auf die Patenterteilung: **08.12.1999**
(21) Anmeldenummer: 95113608.4
(22) Anmeldetag: 30.08.1995
(51) Int. Cl.: A61K 9/127

(54) **Injizierbare liposomale Arzneizubereitungen**
Injectable liposomal compositions
Compositions liposomiques injectables

(30) Priorität: 12.09.1994 DE 4432378
(43) Veröffentlichungstag der Anmeldung: 13.03.1996
(73) Patentinhaber: BAYER AG, 51368 Leverkusen (DE)
(72) Erfinder: Hamann, Hans-Jürgen, Dr., D-41539 Dormagen (DE); Serno, Peter, Dr., D-51467 Bergisch Gladbach (DE); Herboth, Matthias, Dr., D-51375 Leverkusen (DE); Kurka, Peter, Dr., D-40723 Hilden (DE)

(56) Entgegenhaltungen:
- EP-A- 0 200 068
- EP-A- 0 560 138
- THE JOURNAL OF MEMBRANE BIOLOGY, Bd. 33, 1977 NY, USA, Seiten 157-201, MAHENDRA KUMAR JAIN ET AL 'Effect of Small Molecules on the Dipalmitoyl Lecithin Liposomal Bilayer: III. Phase Transition in Lipid Bilayer'
- INTERNATIONAL JOURNAL OF PHARMACEUTICS, Bd. 4, 1980 NETHERLANDS, Seiten 281-290, N. MURANUSHI ET AL 'Mechanism for the inducement of the intestinal absorption of poorly absorbed drugs by mixed micelles II. Effect of the incorporation of various lipids on the permeability of liposomal membranes'
- DATABASE MEDLINE US NATIONAL LIBRARY OF MEDICINE (NLM), BETHESDA, MD, US AN: 89134809, CROWE J H ET AL 'Effects of free fatty acids and transition temperature on the stability of dry liposomes'

## Beschreibung

Die Erfindung betrifft stabile liposomale injizierbare Formulierungen für lipophile, schwerlösliche Wirkstoffe in Form von Liposomen, die durch kurzkettige Fettsäuren stabilisiert werden, insbesondere für Wirkstoffe aus der Gruppe der Dihydropyridine.

Für viele wertvolle Arzneiwirkstoffe werden intravenös applizierbare Formulierungen benötigt, z. B. für eine schnelle Initialtherapie, für akute Notfallsituationen und auch zur Behandlung schwerer klinischer Fälle. Viele Wirkstoffe, einschließlich vieler Dihydropyridine, zeigen eine sehr schlechte Wasserlöslichkeit und sind somit in wässrigen Lösungen nicht formulierbar. Ein weiteres Problem ist die Hydrolyse- und Oxidationsempfindlichkeit vieler Substanzen.

Das Problem der Löslichkeit und der Hydrolysempfindlichkeit kann durch die Verwendung von üblichen organischen Lösungsmitteln, wie Ethanol, Polyethylenglycol oder Propylenglycol, gelöst werden. Viele dieser Lösungsmittel zeigen jedoch eine schlechte lokale Verträglichkeit und müssen daher über einen Zentralkatheter appliziert oder mit wässrigen Infusionslösungen verdünnt werden. Letzteres kann aufgrund schlechter Wasserlöslichkeit zu Übersättigungen und zu Kristallisationserscheinungen führen. Bei längerer intravenöser Therapie sind die dabei zu verabreichenden Mengen an organischen Lösungsmitteln oft toxikologisch bedenklich. Die Verwendung von solubilisierenden Seifen- oder Tensidmizellen, die durch Einarbeiten des Wirkstoffs in wässrigen Natriumlaurylsulfat- oder Polysorbatlösungen formuliert werden können, ist ebenfalls problematisch, da hierdurch schwere Komplikationen, wie z. B. Hämolyse oder schockartige Symptome, hervorgerufen werden können.

Es sind zahlreiche Verfahren bekannt, die schwer lösliche Wirkstoffe in nanopartikuläre Träger inkorporieren und dabei weitgehend auf den Einsatz toxischer Lösungsmittel und Tenside verzichten. Hierzu gehören z. B. die parenteralen Emulsionen, Lecithin-Gallensalz-Mischzellen und Liposomen. Bei diesen Systemen liegen die Wirkstoffe als kolloidale Partikel in Wasser dispergiert vor, so daß nur hydrolysestabile Wirkstoffe eingearbeitet werden können.

In der Publikation von N. Muranushi et al., International Journal of Pharmaceutics, 4 (1980), page 281-290, werden Liposomen zur gastrointestinalen Adsorption beschrieben, die wasserlösliche Wirkstoffe enthalten und sich von den erfindungsgemäßen paranteralen Zubereitungen für schwer lösliche lipophile Arzneistoffe eindeutig unterscheiden. In dieser Literatur wird ebenso wie in der Publikation von Mahendra Kumar Jain et al, The Journal of Membrane Biology, 34 (1977), page 157-201, ausdrücklich darauf hingewiesen, daß durch Zugabe kurzkettiger Fettsäuren eine erhöhte Durchlässigkeit der Liposomenmembran auftritt. Bei diesem Stand der Technik war nicht zu erwarten, daß die Verwendung von kurzkettigen Fettsäuren zu einer Stabilisierung der erfindungsgemäßen Liposomen führt.

Es ist ebenfalls bekannt, daß Liposomen mit den darin eingearbeiteten Wirkstoffen durch Gefriertrocknung stabilisiert werden können (vergl. Betageri et al., Liposome Drug Delivery Systems, Technomic Publishing AG Basel, 1993, Seite 118). Dazu ist der Zusatz eines Kryoprotektors notwendig, der aufgrund seiner membranstabilisierenden Wirkung die Integrität der Liposomen gewährleistet. Bekannte Kryoprotektors sind Polyalkohole wie z. B. Glycerin, Monosaccharide wie Glucose, Disaccharide wie z. B. Saccharose, Lactose oder Treholose und Proteine bzw. Aminosäuren (vergl. Y. Ötzer et al., Influence of Freezing and Freeze-drying on the Stability of Liposomes Dispersed in Aqueous Media, Acta Pharm. Technol., 34 (3), 1988, S. 129-139).

Aus EP-A-560 138 sind auch schon stabile, gefriergetrocknete liposomale Zubereitungen mit Dihydropyridinen, z. B. mit Nimodipin, bekannt. Die Herstellung dort erfolgt unter Verwendung von Phospholipiden, üblichen Kryoprotektoren und eines pH-Stabilisators.

Es zeigt sich jedoch, daß bestimmte problematische Wirkstoffe, die sowohl schwer wasserlöslich als auch hydrolyse- und oxidationsempfindlich sind, nach den bisher bekannten Methoden nicht in ausreichend stabile Liposomenformulierungen überführt werden können. Dies gilt insbesondere für Dihydropyridine wie Nimodipin oder solche der allgemeinen Formel (I) 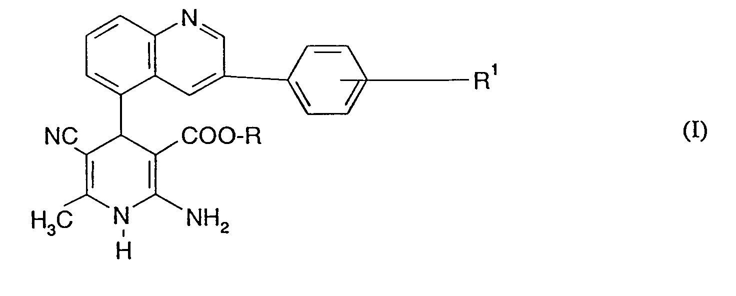 in welcher
R für Alkyl mit 1 - 6 Kohlenstoffatomen steht und
R¹ für Wasserstoff, Halogen, Cyano, Difluormethyl, Alkyl oder Alkoxy mit jeweils 1 - 4 Kohlenstoffatomen steht.

Besonders geeignet für die erfindungsgemäßen Liposomen sind Nimodipin oder Dihydropyridine der allgemeinen Formel I, in welcher
R für Alkyl mit 1 - 4 C-Atomen, insbesondere für n-Propyl oder Iso-Propyl, steht und
R¹ für Wasserstoff, Fluor, Chlor, Cyano oder Trifluormethyl, insbesondere für Wasserstoff steht.

Die Herstellung dieser Dihydropyridine erfolgt nach üblichen Methoden, z. B. nach den in der DOS 4 117 750 beschriebenen Verfahren.

Die Dihydropyridine sind hochwirksame Arzneistoffe, die u. a. zur Therapie der Herzmuskelinsuffizienz eingesetzt werden können. Für diese Indikation ist eine stabile und schnell wirkende i.v.-Formulierung von besonderem Interesse.

Die Erfindung betrifft somit parenteral applizierbare stabile Arzneizubereitungen auf Basis von Liposomen mit Phospholipidmembranen, dadurch gekennzeichnet, daß sie als Stabilisator gegen Ausflockung eine kurzkettige Fettsäure der Formel II 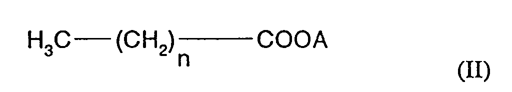 in welcher
n für 4 - 8, insbesondere für 6 steht, und
A für Wasserstoff oder ein 1- oder 2- wertiges Kation, insbesondere Natrium oder Kalium, steht,
   enthalten, wobei das Gewichtsverhältnis Wirkstoff zu Phospholipid 1 zu 20 bis 200 beträgt und das Gewichtsverhältnis kurzkettige Fettsäure zu Phospholipid 1 zu 2 bis 60 beträgt.
Von besonderem Interesse sind liposomale Zubereitungen, in denen das Gewichtsverhältnis kurzkettige Fettsäure zu Phospholipid 1 zu 4 bis 50 beträgt.

Bei dem Versuch, Dihydropyridinwirkstoffe der allgemeinen Formel I gemäß EP-A-560 138 in Liposomen zu überführen, zeigt sich, daß diese sowohl chemisch als auch physikalisch nicht ausreichend stabil waren (siehe Vergleichsbeispiel aus A). Nach einer kurzfristigen Lagerung von nur 2 Wochen bei Raumtemperatur war die Gesamtmenge an unerwünschten Abbauprodukten nicht mehr tolerierbar. Zudem zeigte sich eine ausgeprägte Flockung und Aggregation nach Rekonstitution der Liposomen mit destilliertem Wasser.

Überraschenderweise wurde gefunden, daß man chemisch und physikalisch stabile Liposomen auch mit den problematischen Dihydropyridinen der allgemeinen Formel I erhält, wenn man kurzkettige Fettsäuren der allgemeinen Formel II oder deren Salze zu den Liposomen zugibt.

Der Effekt, daß die erfindungsgemäßen Liposomen durch Zusatz von kurzkettigen Fettsäuren bzw. deren Salzen eine höhere physikalische Stabilität besitzen und die unerwünschte Flockung und Aggregation nach Rekonstititution verhindert wird, konnte bei Kenntnis des Standes der Technik nicht erwartet werden. In der Literatur (Vergl. J. H. Crowe et al, Effects of Free Fatty Acids and Transition Temperature on the Stability of Dry Liposomes, Biochemica et Biophysica Acta, 979, (1989) S. 7-10) wird berichtet, daß übliche Fettsäuren membrandestabilisierend und fusogen, d. h. den Zusammenschluß von Liposomen fördernd, bewirken werden. Durch mikroskopische Untersuchungen, Gelchromatographie und Laserkorrelationsspektroskopie kann gezeigt werden, daß der Zusatz von mittelkettigen Fettsäuren im Bereich der erfindungsgemäßen Konzentration die Partikelgröße der Liposomen nicht signifikant beeinflußt, Flockung und Aggregation der Liposomen verhindert und keine Destasbilisierung bewirkt. Gleichzeitig konnte gezeigt werden, daß die Dihydropyridinwirkstoffe vollständig (100 %) in den Liposomen inkorporiert vorliegen.

Die erfindungsgemäße Konzentration der kurzkettigen Fettsäuren beträgt 0,4 bis 10 mg pro ml der applikationsfertigen Liposomen-Dispersion. Bezogen auf die Menge des eingesetzten Phospholipids wird ein Gewichtsteil der kurzkettigen Fettsäure bzw. ihrer Salze mit 2 bis 60, vorzugsweise 4 bis 50 Gewichtsteile Phospholipid eingesetzt.

Vorteilhaft ist die Zugabe weiterer Hilfsstoffe, insbesondere von Disaccariden wie Trehalose, Saccharose und Lactose als Cryoprotektoren. Insbesondere Saccharose zeigt eine optimale Cryoprotektionswirkung. Das Cryoprotektor-Phospholipid-Verhältnis liegt zwischen 0,8 bis 4 zu 1, vorzugsweise bei 1,2 bis 2,5 zu 1.

Das Wirkstoff-Phospholipid-Verhältnis beträgt 1 zu 20 bis 200, vorzugsweise 1 zu 30 bis 80, insbesondere 1 zu 30 bis 50.

Zur Erzielung eines isotonischen Drucks können dem Rekonstitutionsmedium auch ein oder mehrere geeignete osmotisch aktive Agentien zugesetzt werden. Als geeignet erweisen sich Glycerol, Mannit und Glukose; insbesondere Glycerol.

Erforderlichenfalls können die erfindungsgemäßen Liposome weitere Hilfsstoffe enthalten, wie zum Beispiel Stabilisatoren wie Antioxydantien vom Typ Butylhydroxyanisol, Butylhydroxytoluol, alpha-Tocopherol und deren Salze und Ascorbinsäure und deren Salze und Ester, vorzugsweise Ascorbinsäure und deren Salze, sowie pH-Wert-regulierende Agentien wie Puffer, Säuren oder Basen, insbesondere Ascorbinsäure und Natriumhydroxyd.

Erfindungsgemäß lassen sich übliche Phospholipide einsetzen. Bevorzugt werden Phospholipide verwendet, die nach außen ungeladene Phosphoglyceride darstellen, die gegebenenfalls intramolekulare Zwitterionen sind und der allgemeinen Formel III entsprechen 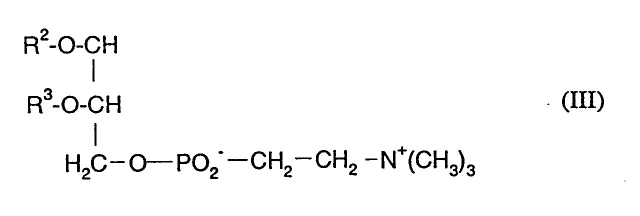 in welcher R² und R³ gleich oder verschieden sind und jeweils gesättigte oder ungesättigte Acylgruppen mit 8 bis 24 C-Atomen darstellen, die gegebenenfalls verzweigt und/oder substituiert sein können.
Neben den Phospolipiden der Formel III können auch in kleineren Mengen andersartige Phospholipide wie Phosphatidylethanolamine, Phosphatidylinositole, Sphingomyeline, Phosphatidylglycerole und/oder Phosphatidsäuren erhalten werden. Die eingesetzten Phospholipide können durch Aufreinigung aus natürlichen Quellen wie Soja- oder Eirohlecithin oder synthetisch hergestellt werden. Bevorzugt werden gereinigte Eilecithine eingesetzt.

Die erfindungsgemäßen Liposomen können nach üblichen Herstellungsverfahren erhalten werden, zum Beispiel durch Hochdruckhomogenisation, Porenextrusion, Dialyse- und Verdünnungsverfahren und Ultraschalldispergierung, vorzugsweise durch Hochdruckhomogenisation, wie zum Beispiel Mikrofluidisation.

Die Erfindung betrifft somit auch Verfahren zur Herstellung der erfindungsgemäßen Liposome, dadurch gekennzeichnet, daß man ein Gewichtsteil des lipophilen Wirkstoffs und 20 bis 200 Gewichtsteile Phospholipid gegebenenfalls zusammen mit einem Antioxydans bei einer Temperatur zwischen 10 und 90°C, vorzugsweise zwischen 50 und 80°C in Wasser mit einem Rührwerk vordispergiert, gegebenenfalls unter Stickstoffbegasung, und dann in einem Hochdruckdüsenhomogenisator bei Temperaturen zwischen 20 und 80°C und einem Druck zwischen 400 und 1500 bar auf eine mittlere Teilchengröße von 35 bis 200 nm homogenisiert und anschließend einen Cryoprotektor und eine kurzkettige Fettsäure der allgemeinen Formel II oder ihr Salz in einem Fettsäure-Phospholipid-Verhältnis von 1 zu 2 bis 60 im Homogenisat löst und die so erhaltene Dispersion gefriergetrocknet.

Bei einer Variante dieses Verfahrens können der Wirkstoff und/oder der Cryoprotektor auch bereits während der Vordispersion oder der Hochdruckdüsenhomogenisation zugesetzt werden. Ebenso lassen sich die kurzkettigen Fettsäuren auch dem Rekonstitutionsmedium zusetzen, so daß sie erst während des Rekonstitutionsvorganges mit den Liposomen direkt in Kontakt kommen. In beiden Fällen wird eine Flockung der rekonstituierten Liposomen über mehr als 24 Stunden verhindert, und gleichzeitig bleibt der mittlere Partikeldurchmesser der Liposomen erhalten und die chemische Stabilität des Wirkstoffs gewährleistet.

Bei einer Überschreitung der Konzentration der Fettsäuren von 10 mg/ml tritt eine Destabilisierung der Liposomen und ein Austritt des Wirkstoffes aus den Liposomen auf, wie aus Vergleichsbeispiel B ersichtlich ist. Ohne den erfindungsgemäßen Zusatz von kurzkettigen Fettsäuren flocken die Dispersionen innerhalb von 24 Stunden aus, wie aus Vergleichsbeispiel C ersichtlich ist.

Die nachfolgenden Ausführungsbeispiele zeigen für typische Wirkstoffe exemplarisch die Vorteile des erfindungsgemäßen Verfahrens und die Stabilität der so erhaltenen Liposomen. Die Vergleichsbeispiele A-C zeigen dagegen die Instabilität und die Nachteile der Produkte, die nach bisher bekannten Verfahren hergestellt wurden, bzw. die außerhalb der beanspruchten Formulierungen liegen. Alle Prozentangaben sind Gewichtsprozente.

### Ausführungsbeispiele

### Ausführungsbeispiel 1:

5,5997 kg dest. Wasser werden 30 min. mit Stickstoff begast. Nach der Begasung werden 16,2 g Natriumascorbat im Wasser gelöst und anschließend 580,5 g gereinigtes Eilecithin (Phosphatidylcholin > 94%) zugegeben. Diese Mischung wird 30 min. mit einem schnellaufenden Rührer bei 65°C dispergiert. Nach Ergänzung des verdunsteten Wassers wird die Vordispersion durch einen Membranfilter (Porengröße 8µm) filtriert und in einen Hochdruckhomogenisator überführt.

Die Dispersion wird 5 Durchgänge bei 800 bar und 65°C homogenisiert. Daraufhin werden 145 g des Wirkstoffs 2-Amino-1,4-dihydro-5-cyano-6-methyl-4-(3-phenylchinolin-5-yl)pyridin-3-carbonsäureisopropylester zugesetzt und bei niedrigem Druck (25 bar) in der Dispersion gleichmäßig verteilt. Anschließend werden 20 Durchgänge bei 65°C und 800 bar homogenisiert. Die Liposomen werden auf Raumtemperatur abgekühlt.

Pro Gramm Homogenisatausbeute werden 168,2 mg Saccharose und 0,11mg Ascorbinsäure zugesetzt und gelöst. Die Dispersion wird mit 0,1 N NaOH-Lösung auf pH 6,5 eingestellt und durch einen Membranfilter (0,2 µm Porengröße) sterilfiltriert, zu je 2,5 ml in Braunglasfläschchen abgefüllt und gefriergetrocknet.

Die mittlere Liposomengröße vor Gefriertrocknung beträgt 48 nm. Nach Rekonstitution mit 10 ml einer wäßrigen Lösung mit 0,0495 % Natriumcaprylat und 1,51 % Glycerol beträgt die mittlere Liposomengröße 57 nm. Nach 24-stündiger Standzeit der rekonstituierten Lösung beträgt die mittlere Liposomengröße 57 nm. Innerhalb von 24 Stunden tritt in der rekonstituierten Dispersion keine Flockung und/oder Niederschlagsbildung auf.

Gelchromatographische Untersuchungen zeigen, daß der Wirkstoff zu 100 % in den Liposomen inkorporiert vorliegt.

### Ausführungsbeispiel 2:

In 638,33 g dest. Wasser, das zuvor 10 min. mit Stickstoff begast worden ist, wird 1,8561 g Natriumascorbat gelöst. Dieser Lösung werden 66 g gereinigtes Eilecithin (Phosphatidylcholingehalt > 94 %) zugegeben. Die Mischung wird mit einem schnellaufenden Rührer 30 min. bei 60°C dispergiert. Anschließend wird mit stickstoffbegastem Wasser auf 706,2 g aufgefüllt und 1,65 g des Wirkstoffs gemäß Ausführungsbeispiel 1 zugesetzt. Um den Wirkstoff gleichmäßig zu verteilen wird nochmals 3 min. dispergiert.

Die Dispersion wird in einen Hochdruckhomogenisator überführt und 25 Passagen bei 60°C und 800 bar homogenisiert.

Anschließend werden in der Liposomendispersion 637,19 g Saccarose gelöst und der pH-Wert der Dispersion mit 0,1-N NaOH-Lösung auf pH 6,5 eingestellt.

Nach Sterilfiltration (Membranfilter 0,2 µm Porengröße) wird die fertige Dispersion zu je 2,64 g in Braunglasfläschchen gefüllt und gefriergetrocknet.

Nach dreimonatiger Lagerung der gefriergetrockneten Liposomen bei 40°C beträgt der Wirkstoffgehalt 98,5 % und innerhalb von 24 Stunden nach Rekonstitution tritt keine Flockung und/oder Niederschlagsbildung auf mit 10 ml einer wäßrigen Lösung mit 0,0495 % Natriumcaprylat und 1,51 % Glycerol.

### Ausführungsbeispiel 3:

12,53 g Ascorbinsäure und 2,85 g Natriumhydroxid werden in 4868,18 g Wasser unter Rühren gelöst und anschließend 504,8 g gereinigtes Eilecithin (Phosphatidylcholin > 94 %) zugegeben und diese Mischung in einem schnell laufenden Mixer dispergiert. Die Dispersion wird durch ein Membranfilter (Porengröße 8 µm) filtriert und dann in einem Hochdruckhomogenisator bei 800 bar in 25 Durchgängen homogenisiert. Daraufhin werden 908,70 g Sucrose in der Liposomendispersion gelöst und der pH-Wert auf 6,5 eingestellt bei Lösen von 0,6 g Ascorbinsäure und entsprechendem Titrieren mit 0,1 N Natriumhydroxidlösung. Zum Schutz vor Oxidation wird das gesamte Verfahren unter Stickstoffgas durchgeführt.

12,6g(4R)Isopropyl-2-methoxyethyl-4-(2-chlor-3-cyano-phenyl)-1,4-dihydro-2,6-dimethyl-pyridin-3,5-dicarboxylat werden zugegeben und die Dispersion 12 Stunden gerührt bis die gesamte Menge des Wirkstoffs gelöst ist. Die Dispersion wird nach erneuter Filtration in Portionen von 11,88 ml in 50 ml Fläschchen gefriergetrocknet. Das lyophilisierte Produkt wird rekonstituiert mit einer Lösung von 0,725 g Glycerin und 0,02375 g Natriumcaprylat in 47,184 g destilliertem Wasser.

### Ausführungsbeispiel 4:

4,6583 kg destilliertes Wasser wird für 10 Minuten mit Stickstoff begast und anschließend werden 5,7 g Ascorbinsäure und 852,8 g Glucose darin gelöst. Die Lösung wird auf pH 6,5 eingestellt durch Einsatz von 66 g einer 0,5 molaren Argininlösung. Danach werden 9,502 g des Wirkstoffs Nimodipin und 473,8 g gereinigtes Eilecithin (Phosphatidylcholin > 80 %) zugegeben. Diese Mischung wird bei 75°C für 60 Minuten mit einem schnell laufenden Rührer dispergiert unter Stickstoffatmosphäre. Die Dispersion wird gefiltert (Porengröße 5 µm) und dann in einem Hochdruckhomogenisator bei 800 bar und 75°C homogenisiert und anschließend auf unter 30°C abgekühlt. Die Dispersion wird steril filtriert durch Membranfilter (Porengröße 0,2 µm) und jeweils 15,30 ml in braune Glasfläschchen gefüllt und gefriergetrocknet.

Die Liposomen werden rekonstituiert mit einer Lösung von 0,0495 % Natriumcaprylat und 1,513 % Glycerin in Wasser. Für mindestens 24 Stunden nach Rekonstitution zeigt die Dispersion weder einen Niederschlag noch eine Ausflockung.

### Ausführungsbeispiel 5:

171,6 g destilliertes Wasser werden für 20 Minuten mit Stickstoff begast und anschließend werden 200 mg Natriumascorbat und 10 g gereinigtes Eilecithin (Phosphatidylcholingehalt > 94 %) und 200 mg des Wirkstoffs Etomidat ((R)-Ethyl-(α-methylbenzyl)-5-imidazolcarboxylat) zugegeben. Die Mischung wird für 20 Minuten bei 60°C unter Stickstoffatmosphäre in einem Hochgeschwindigkeitsrührer dispergiert. Nach Ersatz des verdampften Wassers wird die Prädispersion in einen Hochdruckhomogenisator gegeben und in 25 Passagen bei 800 bar und 60°C homogenisiert. Anschließend werden 98,9 mg Sucrose und 1,8 mg Natriumcaprylat pro g Homogerisat zugefügt und gelöst und der pH-Wert der Dispersion auf 6,5 eingestellt unter Verwendung von 1 N Natronlauge. Nach Sterilfiltration (Membranfilter 0,2 µm Porengröße) wird die Dispersion zu 20,0 g in braune Glasfläschchen gefüllt und gefriergetrocknet.

Die mittlere Liposomengröße vor der Gefriertrocknung beträgt 56 nm. Nach Rekonstitution mit 17,16 g Glucoselösung (5 %ig) beträgt die mittlere Liposomengröße 61 nm. Für mindestens 4 Stunden nach der Rekonstitution ist weder eine Ausflockung noch ein Niederschlag festzustellen.

### Ausführungsbeispiel 6:

1,4 g Natriumascorbat werden in 425 g sauerstofffreiem destilliertem Wasser gelöst. Dann werden 50 g gereinigtes Eilecithin (Phosphatidylcholingehalt > 80 %) und 1,5 g Paclitaxel (Taxol) zugegeben und anschließend mit einem schnell laufenden Rührer bei 65 °C für 30 Minuten dispergiert. Die erhaltene Dispersion wird in einem Hochdruckhomogenisator bei 725 bar und 60°C über 25 Passagen homogenisiert.

Zu 382,32 g dieser homogenisierten Dispersion werden 72 g Sucrose und 80 mg Ascorbinsäure gegeben, der pH-Wert wird durch 0,1 N Natriumhydroxidlösung auf 6,5 eingestellt und die Dispersion mit Wasser auf 480 g aufgefüllt. Anschließend wird die Dispersion gefiltert und zu Portionen von 12,32 g (entspricht 30 mg Paclitaxel + 2,67 % Überfüllung) in 50 ml Fläschchen gefüllt und gefriergetrocknet. Das gefriergetrocknete Produkt wird rekonstituiert mit 29,7 g einer Lösung enthaltend 0,725 g Glycerin und 0,02375 g Natriumcaprylat in 47,187 g destilliertem Wasser. 30 ml dieser rekonstituierten Dispersion enthalten 30 mg Paclitaxel.

### Vergleichsbeispiele

### Vergleichsbeispiel A: (gemäß EP-A-560 138)

In 253 g dest. Wasser, das zuvor 10 min. mit Stickstoff begast worden ist, werden 49,1 g Glucose und 0,345 g Ascorbinsäure gelöst. Anschließend wird der pH-Wert mit 3,9 g 0,5-M-Argininlösung auf pH 6,5 eingestellt. Dieser Lösung werden 27,25 g gereinigtes Eilecithin (Phosphatidylcholingehalt > 80%), 0,345 g des Wirkstoffs gemäß Ausführungsbeispiel 1 und stickstoffbegastes dest. Wasser bis zu einem Gesamtgewicht von 345 g zugesetzt. Die Mischung wird 30 min. mit einem schnellaufenden Rührwerk bei 75°C dispergiert. Nach Filtration (Membranfilter 5 µm Porengröße) wird die Dispersion in einen Hochdruckhomogenisator überführt und 25 Passagen bei 800 bar und 75°C homogenisiert.
Die fertige Liposomendispersion wird sterilfiltriert (Membranfilter 0,2 µm Porengröße), zu je 2,1 ml in Braunglasfläschchen abgefüllt und gefriergetrocknet.

Nach zweiwöchiger Lagerung der gefriergetrockneten Liposomen bei 40°C fällt der Wirkstoffgehalt auf 89,7 %.

Der mittlere Partikeldurchmesser der Liposomen nach Hochdruckhomogenisation beträgt 45 nm. Nach Rekonstitution der gefriergetrockneten Liposomen mit 9,5 ml dest. Wasser beträgt der mittlere Liposomendurchmesser 47 nm. Innerhalb von 24 Stunden nach Rekonstitution flockt die Liposomendispersion aus und bildet einen Niederschlag.

### Vergleichsbeispiel B: (zu viel Fettsäure)

Die nach Ausführungsbeispiel 1 hergestellten Liposomen werden mit 10 ml einer wäßrigen Lösung mit 5 % Natriumcaprylat und 1,51 % Glycerol rekonstituiert. Die Liposomen werden destabilisiert. Nur noch 5,3 % des Wirkstoffs liegt in Liposomen inkorporiert vor.

### Vergleichsbeispiel C: (keine Fettsäure)

Die nach Ausführungsbeispiel 1 hergestellten Liposomen werden mit 10 ml einer wäßrigen Lösung mit 1,51 % Glycerol rekonstituiert. Die Dispersion flockt innerhalb von 24 Stunden aus und bildet einen Niederschlag.

## Patentansprüche

1. Parenteral applizierbare, stabile Arzneizubereitungen für lipophile, schwerlösliche Wirkstoffe in Form von Liposomen mit Phospholipidmembranen, wobei das Wirkstoff/Phospholipidgewichtsverhältnis 1 zu 20 bis 200 beträgt und der mittlere Liposomendurchmesser zwischen 35 und 200 nm liegt, dadurch gekennzeichnet, daß sie als Stabilisator eine kurzkettige Fettsäure der allgemeinen Formel II enthalten 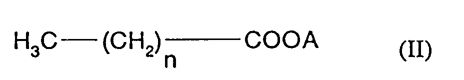 in welcher
n für 4 bis 8 steht und
A für Wasserstoff oder ein ein- oder zweiwertiges Kation steht und die Konzentration der kurzkettigen Fettsäure in der applikationsfertigen Lösung zwischen 0,3 und 10,0 mg/ml beträgt.

2. Arzneizubereitung gemäß Anspruch 1, wobei das Gewichtsverhältnis der kurzkettigen Fettsäuren bzw. ihrer Salze zu dem Phospholipid 1 zu 2 bis 60 beträgt.

3. Arzneizubereitung gemäß Anspruch 1 enthaltend als Wirkstoff ein Dihydropyridin.

4. Arzneizubereitung gemäß Anspruch 1 enthaltend als Wirkstoff Nimodipin oder ein Dihydropyridin der Formel I 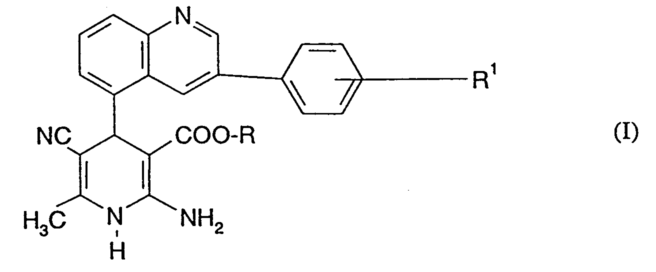 in welcher
R für Alkyl mit 1 - 6 Kohlenstoffatomen steht und
R¹ für Wasserstoff, Halogen, Cyano, Difluormethyl, Alkyl oder Alkoxy mit jeweils 1 - 4 Kohlenstoffatomen steht.

5. Arzneizubereitung gemäß Anspruch 4 enthaltend als Wirkstoff ein Dihydropyridinder Formel I, in welcher
R¹ für Wasserstoff, Fluor, Chlor, Cyano oder Trifluormethyl steht und
R für Alkyl mit 1 - 4 C-Atomen steht.

6. Verwendung von kurzkettigen Fettsäuren der allgemeinen Formel II 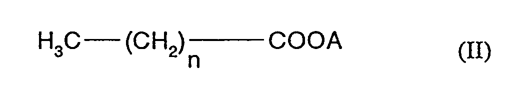 in welcher
n für 4 - 8 steht und
A für Wasserstoff oder ein 1- oder 2- wertiges Kation steht bei der Herstellung von parenteral applizierbaren stabilen Arzneizubereitungen für lipophile, schwerlösliche Wirkstoffe in Form von Liposomen mit Phospholipidmembranen.

7. Arzneizubereitungen gemäß Anspruch 1, dadurch gekennzeichnet, daß sie als Cryoprotektoren Disaccharide enthalten.

8. Arzneizubereitungen gemäß Anspruch 1, dadurch gekennzeichnet, daß sie als Cryoprotektoren 0,8 bis 4,0 Gewichtsteile Saccharose bezogen auf ein Gewichtsteil Phospholipid enthalten.

## Claims

1. Parenterally administrable, stable pharmaceutical preparations for lipophilic, poorly soluble active compounds, in the form of liposomes with phospholipid membranes, the active compound/phospholipid weight ratio being 1 to 20 - 200 and the average liposome diameter being between 35 and 200 nm, characterized in that they contain, as a stabilizer, a short-chain fatty acid of the general formula II 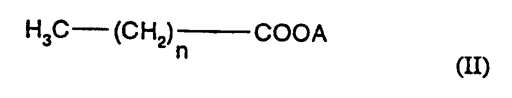 in which
n represents 4 to 8 and
A represents hydrogen or a mono- or divalent cation and the concentration of the short-chain fatty acid in the ready-to-administer solution is between 0.3 and 10.0 mg/ml.

2. Pharmaceutical preparation according to Claim 1, the weight ratio of the short-chain fatty acids or their salts to the phospholipid being 1 to 2 - 60.

3. Pharmaceutical preparation according to Claim 1 containing a dihydropyridine as active compound.

4. Pharmaceutical preparation according to Claim 1 containing as active compound nimodipine or a dihydropyridine of the formula I 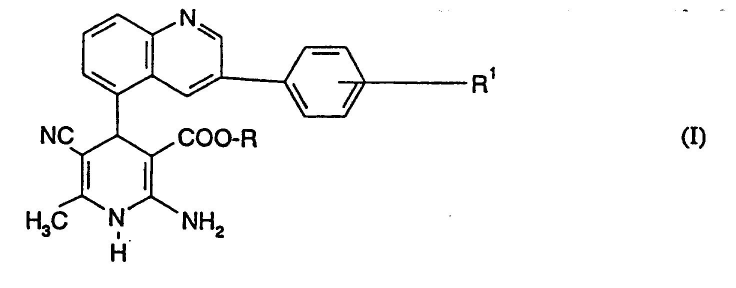 in which
R represents alkyl having 1 - 6 carbon atoms and
R¹ represents hydrogen, halogen, cyano, difluoromethyl, or alkyl or alkoxy each having 1 - 4 carbon atoms.

5. Pharmaceutical preparation according to Claim 4 containing as active compound a dihydropyridine of the formula I, in which
R¹ represents hydrogen, fluorine, chlorine, cyano or trifluoromethyl and
R represents alkyl having 1 - 4 C atoms.

6. Use of short-chain fatty acids of the general formula II 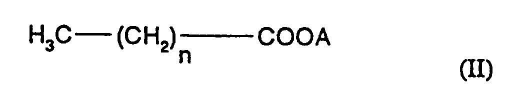 in which
n represents 4 - 8 and
A represents hydrogen or a 1 - or 2-valent cation, in the production of parenterally administrable stable pharmaceutical preparations for lipophilic, poorly soluble active compounds in the form of liposomes having phospholipid membranes.

7. Pharmaceutical preparations according to Claim 1, characterized in that as cryoprotectors they contain disaccharides.

8. Pharmaceutical preparations according to Claim 1, characterized in that as cryoprotectors they contain 0.8 to 4.0 parts by weight of sucrose, relative to one part by weight of phospholipid.

## Revendications

1. Compositions pharmaceutiques stables, administrables de manière parentérale, pour agents actifs lipophiles, difficilement solubles, sous forme de liposomes à membrane phospholipidique, où le rapport pondéral agent actif/phospholipide se situe dans l'intervalle allant de 1/20 à 200 et le diamètre moyen des liposomes se situe dans l'intervalle allant de 35 à 200 nm, caractérisées en ce qu'elles contiennent comme stabilisants, un acide gras à chaîne courte de formule générale (II) :
H₃C - (CH₂)ₙ - COOA II
dans laquelle :
n représente 4 à 8, et
A représente hydrogène ou un cation mono- ou divalent, et en ce que
la concentration de l'acide gras à chaîne courte dans la solution prête à être administrée se situe dans l'intervalle allant de 0,3 à 10,0 mg/ml.

2. Compositions pharmaceutiques suivant la revendication 1, où le rapport pondéral de l'acide gras à chaîne courte ou de son sel aux phospholipides se situe dans l'intervalle allant de 1/2 à 60.

3. Compositions pharmaceutiques suivant la revendication 1, contenant une dihydropyridine comme agent actif.

4. Compositions pharmaceutiques suivant la revendication 1, contenant comme agent actif, la nimodipine ou une dihydropyridine de formule (I) : 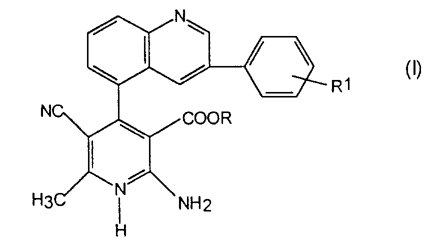 dans laquelle :
R représente alcoyle ayant 1 à 6 atomes de carbone, et
R¹ représente hydrogène, halogène, cyano, difluorométhyle, alcoyle ou alcoxy ayant chaque fois, 1 à 4 atomes de carbone.

5. Compositions pharmaceutiques suivant la revendication 4, contenant comme agent actif, une dihydropyridine de formule (I), dans laquelle :
R¹ représente hydrogène, fluor, chlore, cyano ou trifluorométhyle, et
R représente alcoyle ayant 1 à 4 atomes C.

6. Utilisation d'acides gras à chaîne courte de formule générale (II) :
H₃C - (CH₂)ₙ - COOA (II)
dans laquelle :
n représente 4 à 8, et
A représente hydrogène ou un cation mono- ou divalent,
dans la préparation de compositions pharmaceutiques stables, administrables de manière parentérale, pour agents actifs lipophiles, difficilement solubles, sous forme de liposomes à membrane phospholipidique.

7. Compositions pharmaceutiques suivant la revendication 1, caractérisées en ce qu'elles contiennent un disaccharide comme cryoprotecteur.

8. Compositions pharmaceutiques suivant la revendication 1, caractérisées en ce qu'elles contiennent comme cryoprotecteur, de 0,8 à 4,0 parties en poids de saccharose, sur base d'une partie en poids de phospholipide.
